# EUROPÄISCHE PATENTANMELDUNG

(11) **EP 1 983 024 A1**
(43) Veröffentlichungstag der Anmeldung: **22.10.2008**
(21) Anmeldenummer: 08154150.0
(22) Anmeldetag: 07.04.2008
(51) Int. Cl.: C08K 5/12, C07C 69/76, C07C 67/08, C07C 67/10, C08L 57/08, C08L 21/00

(54) **Stoffgemische auf Basis von Alkylbenzylestern**

(30) Priorität: 21.04.2007 DE 102007018992
(71) Anmelder: LANXESS Deutschland GmbH, 51369 Leverkusen (DE)
(72) Erfinder: Weiß, Thomas, 68549, Ilvesheim (DE); Kuckert, Eberhard, 51375, Leverkusen (DE); Wiedemeier, Melanie, 41540, Dormagen-Delhoven (DE); Hansel, Jan-Gerd, 51469, Bergisch Gladbach (DE)

(57) **Zusammenfassung**

Die vorliegende Erfindung betrifft Estermischungen aus Alkylbenzylestem und Polybenzylestem, insbesondere Trimellitsäurcestern, ein Verfahren zur Herstellung solcher Estermischungen sowie ihre Verwendung als Weichmacher.

## Beschreibung

Die Erfindung betrifft Estermischungen aus Alkylbenzylestern und Polybenzylestern, ein Verfahren zur Herstellung solcher Estermischungen sowie ihre Verwendung als Weichmacher.

Zur Verarbeitung von Kunststoffen wie Polyvinylchlorid werden seit Jahrzehnten Weichmacher eingesetzt. In letzter Zeit steigen die Anforderungen an die Weichmacher in Hinblick auf Leistungsfähigkeit und Unbedenklichkeit gegenüber Mensch und Umwelt. Eine wichtige Forderung betrifft eine möglichst niedrige Lösetemperatur. Unter der Lösetemperatur wird im Zusammenhang mit Weichmachern die Temperatur verstanden, bei der aus einer Polyvinylchlorid-Dispersion in einem Weichmacher eine homogene Phase wird (L. Meier: "Weichmacher", in R. Gächter, H. Müller (Ed.): Taschenbuch der Kunststoffadditive, 3. Ausgabe, S. 361 - S. 362, Hanser Verlag, München 1990). Weichmacher mit niedriger Lösetemperatur ermöglichen eine schnelle und energiesparende Verarbeitung.

In Anwendungen, in denen eine niedrige Lösetemperatur gefordert ist, werden nach dem Stand der Technik überwiegend Alkylbenzylester von aromatischen Polycarbonsäuren, wie beispielsweise das Butylbenzylphthalat, eingesetzt (L. Meier: "Weichmacher", in R. Gächter, H. Müller (Ed.): Taschenbuch der Kunststoffadditive, 3. Ausgabe, S. 397, Hanser Verlag, München 1990). Diese sind kostengünstig durch Umsetzung von aliphatischen Alkoholen mit aromatischen Polycarbonsäuren oder ihren cyclischen Anhydriden und Benzylhalogeniden wie zum Beispiel Benzylchlorid in Gegenwart einer Base herstellbar. Das Herstellverfahren kann schrittweise oder in einem Eintopfverfahren (DE-A 1 468 373 für Benzylallcylphthalate, DE-A 1 593 047 für Benzylalkyltrimellitate) durchgeführt werden.

Neben einer niedrigen Lösetemperatur wird von zeitgemäßen Weichmachern aber auch eine niedrige Flüchtigkeit verlangt. Die Flüchtigkeit von Weichmachern führt zu unerwünschter Versprödung des Weich-Polyvinylchlorid sowie zur Belastung mit sogenannten Flüchtigen Organischen Substanzen (VOC), die in verbrauchernahen Anwendungen vermieden werden soll.

Butylbenzylphthalat weist eine sehr niedrige Lösetemperatur auf, ist allerdings flüchtig. Handelsübliche Trialkyltrimellitate wie z. B. Trioctyltrimellitat oder Uraplast^{®} 525 (C₈-/C₁₀-Trialkyltrimellitat von linearen C₈- bis C₁₀- Alkoholen) weisen hohe Lösetempearturen aber niedrige Flüchtigkeit auf.

Die Herstellung von n-Butyldibenzyltrimellitat durch Veresterung einer Mischung aus Trimellitsäureanhydrid, n-Butanol und Benzylchlorid in Gegenwart von stöchiometrischen Mengen Triethylamin und dessen Verwendung als Weichmacher wird in DE-A 1 593 047 beschrieben. Die Verwendung stöchiometrischer Mengen Triethlyamin bringt Nachteile mit sich. Das in der Reaktion aus dem Triethylamin gebildete Ammoniumsalz gelangt während der wässrigen Aufarbeitung des Reaktionsgemisches in das Abwasser, wo es wegen seiner großen Menge erhebliche Kohlenstoff- und Stickstofffrachten verursacht. Das Abwasser muss daher aufwändig geklärt werden. Weiterhin ist bekannt, dass ein Teil des eingesetzten Benzylchlorids mit dem Triethylamin zu Triethylbenzylammoniumchlorid reagiert, welches ebenfalls im Abwasser endet. Damit geht nicht nur das kostbare Amin vollständig verloren, sondern auch ein Teil des Benzylchlorids.

Die Aufgabe der vorliegenden Erfindung bestand darin, Weichmacher mit niedriger Lösetemperatur und niedriger Flüchtigkeit zu finden, die einfach und ohne Verwendung stöchiometrischer Mengen organischer Amine herstellbar sind.

Überraschend wurde nun gefunden, dass Estermischungen aus Alkylbenzylestern aromatischer Tri- und Tetracarbonsäuren und Polybenzylester aromatischer Tri- und Tetracarbonsäuren unerwartet niedrige Lösetemperaturen aufweisen, dabei eine niedrige Flüchtigkeit besitzen und besonders vorteilhaft herstellbar sind. Gegenstand dieser Erfindung sind Estermischungen enthaltend
a) 80 - 99 Gewichts-% eines oder mehrerer Ester der Struktur (R¹OOC)ₙ-Z-(COOR²)ₚ
b) 1-20 Gewichts-% eines Esters der Struktur (R²OOC)ₘ-Z
wobei die Summe der Gewichts-% aus den Komponenten der Estermischungen 100 ergibt und worin
- R¹: ein geradkettiger oder verzweigter C₁- bis C₂₀-Alkylrest,
- R²: ein optional mit C₁- bis C₄-Alkyl oder Halogen substituierter Benzylrest,
- Z: ein m-wertiger aromatischer C₆- bis C₁₀-Kohlenwasserstoffrest,
- m: eine Zahl von 3 bis 4 und
- n und p: jeweils eine Zahl von 1 bis 3 unter der Maßgabe n + p = m
bedeuten.

Bevorzugt steht R¹ für n-Butyl, 2-Ethylhexyl oder Isononyl.

Bevorzugt steht R² für Benzyl (-CH₂-Ph).

Bevorzugt leitet sich der Rest Z strukturell von der Pyromellitsäure oder der Trimellitsäure ab.

In einer besonders bevorzugten Form der Erfindung handelt es sich bei den Estern der Struktur (R¹OOC)ₙ-Z-(COOR²)ₚ um Butyldibenzyltrimellitat bzw. Dibutylbenzyltrimellitat und bei dem Ester der Struktur (R²OOC)ₘ-Z um Tribenzyltrimellitat.

Die erfindungsgemäßen Estermischungen lassen sich durch Mischen der an sich bekannten Komponenten im angegebenen Verhältnis herstellen. Bevorzugt werden sie aber durch eine Mischveresterung hergestellt. Die Erfindung betrifft daher auch ein Verfahren zur Herstellung von Mischungen von Alkylbenzylestern aromatischer Tri- oder Tetracarbonsäuren mit Polybenzylestem aromatischer Tri- oder Tetracarbonsäuren, dadurch gekennzeichnet, dass mindestens eine aromatische Tri- oder Tetracarbonsäure oder ein Derivat davon, mindestens ein Benzylhalogenid und mindestens ein aliphatischer Alkohol bei Temperaturen von 50 bis 300 °C und bei Drücken von 2 mbar bis 4 bar, in Gegenwart einer anorganischen Base und substöchiometrischer Mengen eines Phasentransferkatalysators miteinander umgesetzt werden.

Das Verfahren kann in einem Schritt oder in zwei Schritten durchgeführt werden. Bei der Durchführung in einem Schritt werden alle Reaktionsteilnehmer im Wesentlichen gleichzeitig miteinander in Kontakt gebracht und umgesetzt. Bei der Durchführung in zwei Schritten werden bevorzugt in einem ersten Schritt die aromatische Tri- oder Tetracarbonsäure oder ein Derivat davon mit dem aliphatischen Alkohol umgesetzt und eine dabei erhaltene Reaktionsmischung in einem zweiten Schritt mit dem Benzylchlorid, der anorganischen Base und dem Phasentransferkatalysator umgesetzt.

Unabhängig davon, ob das Verfahren in einem oder in zwei Schritten durchgeführt wird, kann es in ansatzweiser oder kontinuierlicher Arbeitsweise durchgeführt werden. Die Reaktionsmischung kann mit einem Lösungsmittel verdünnt werden. Der oben beschriebenen Umsetzung können sich dem Fachmann geläufige Reinigungsoperationen, wie zum Beispiel Extraktion, insbesondere wässrige Wäsche, Destillation, Wasserdampfdestillation, Adsorption und/oder Filtration anschließen.

Im Falle des Einsatzes einer Tricarbonsäure betragen die molaren Mengen der Einsatzstoffe bezogen auf 1 mol der Tricarbonsäure beziehungsweise des Tricarbonsäurederivats vorzugsweise 1,7 bis 2,3 mol Benzylchlorid, 0,7 bis 1,3 mol aliphatischer Alkohol, 0,4 bis 2,5 mol anorganische Base und 0,01 bis 0,3 mol Phasentransferkatalysator. Im Falle des Einsatzes einer Tetracarbonsäure betragen die molaren Mengen der Einsatzstoffe bezogen auf 1 mol einer Tetracarbonsäure beziehungsweise des Tetracarbonsäurederivats vorzugsweise 2,7 bis 3,3 mol Benzylchlorid, 0,7 bis 1,3 mol aliphatischer Alkohol, 1,4 bis 3,5 mol anorganische Base und 0,01 bis 0,4 mol Phasentransferkatalysator.

Die aromatische Tri- oder Tetracarbonsäure kann als solche oder in Form eines Derivats, wie zum Beispiel in Form eines Anhydrids, Esters, Säurechlorids in das erfindungsgemäße Verfahren eingesetzt werden. In einer bevorzugten Ausfuhrungsform der Erfindung werden die Tri- oder Tetracarbonsäuren und/oder ihre Anhydride eingesetzt.

Die bei dem erfindungsgemäßen Verfahren verwendeten Reaktionspartner sind wie oben erwähnt (1) aromatische Tri- oder Tetracarbonsäuren oder deren Anhydride, (2) Benzylchloride, (3) Monohydroxy-aliphatische Alkohole, (4) anorganische Basen und (5) Phasentransferkatalysatoren. Nachfolgend erfolgt eine detaillierte Beschreibung dieser Reaktionspartner:

Erfindungsgemäß einzusetzende Tri- oder Tetracarbonsäuren sind vorzugsweise Benzol-1,2,3-tricarbonsäure, Benzol-1,2,4-tricarbonsäure (Trimellitsäure), Benzol-1,2,3,4-tetracarbonsäure, Benzol-1,2,3,5-tetracarbonsäure oder Benzol-1,2,4,5-tetracarbonsäure (Pyromellitsäure) oder die Anhydride der genannten Säuren. Besonders bevorzugte Anhydride sind Trimellitsäureanhydrid und Pyromellitsäureanhydrid. Für die Zwecke des erfindungsgemässen Verfahrens kann das Anhydrid beispielsweise als Schmelze, als Feststoff, wie z. B. in der Form von Flocken, als Lösung oder Dispersion zu einer Reaktionszone zugegeben werden.

Als errfindungsgemäß einzusetzende Benzylhalogenide sind bevorzugt Benzylchlorid, Benzylbromid, Alkyl-substituierte Benzylchloride oder Benzylbromide, sowie Alkoxy-substituierte Benzylchloride oder Halogen-substituierte Benzylchloride zunennen. Besonders bevorzugt werden Benzylchlorid, Methylbenzylchlorid, Ethylbenzylchlorid, Dimethylbenzylchlorid, Methoxybenzylchlorid, Ethoxybenzylchlorid, Chlorbenzylchlorid, Dichlorbenzylchlorid, Trichorbenzylchlorid, Brombenzylchlorid, Dibrombenzylchlorid oder ähnliche eingesetzt.

Erfindungsgemäß einzusetzende Alkohole, sind einwertige aliphatische Alkohole, wie Methylalkohol, Ethylalkohol, Propylalkohol, Isopropylalkohol, Butylalkohole, zum Beispiel n-Butylalkohol und sek. Butylalkohol, Isobutylalkohol, Amylalkohol, Hexylalkohole, zum Beispiel n-Hexylalkohol, 1,4-Dimethylbutylalkohol, n-Heptylalkohol, Octylalkohole, zum Beispiel Isooctylalkohole, n-Octylalkohol, 2-Ethylhexylalkohol, n-Nonylalkohol, Isononylalkohole, Decylalkohole, zum Beispiel n-Decylalkohol, Isodecylalkohol, Dodecylalkohol, Tridecylalkohol, Tetradecylalkohol, Pentadecylalkohol, Cetylalkohol, Octadecylalkohol, und Eicosylalkohol; cycloaliphatische Alkohole, wie Cyclopropylcarbinol, Cyclobutylalkohol, Cyclopentylalkohol, Methylcyclopentylalkohol, Dimethylcyclopentylalkohol, Äthylcyclopentylalkohol, Cyclohexylalkohol, Methylcyclohexylalkohol, Dimethylcyclohexylalkohol und Cyclooctylalkohol; und ungesättigte aliphatische Alkohole, wie Allylalkohol, Crotylalkohol und ähnliche. Darüber hinaus sind die gesamten verschiedenen isomeren Formen dieser Alkohole und Gemische derselben zur Verwendung in dem erfindungsgemäßen Verfahren geeignet. Daneben berührt die Herkunft des Alkohols nicht das Verfahren, und so können beispielsweise aliphatische Alkohole, welche von Ein- oder Zwei-Stufen-Oxoverfahren, aus der Hydratisierung von Olefinen oder aus der katalytischen Dehydrierung von Kokosnussöl herrühren, verwendet werden und sind tatsächlich wegen ihrer Erreichbarkeit wünschenswert.

Erfindungsgemäß einzusetzende anorganische Basen sind Oxide, Hydroxide, Phosphate, Hydrogenphosphate, Carbonate, Hydrogencarbonate und Silikate der Alkali- und Erdalkalimetalle. Vorzugsweise werden Lithiumcarbonat, Lithiumhydrogencarbonat, Natriumcarbonat, Natriumhydrogencarbonat, Kaliumcarbonat, Kaliumhydrogencarbonat, Magnesiumcarbonat, Calciumcarbonat, Magnesiumhydroxid, Calciumhydroxid, Magnesiumoxid oder Calciumoxid eingesetzt. Insbesondere bevorzugt werden die anorganischen Basen in ihrer wasserfreien Form eingesetzt, wie z.B. Natriumcarbonat.

Für die Verwendung in dem erfindungsgemäßen Verfahren sind alle bekannten Phasentransferkatalysatoren geeignet. Bevorzugt ist der Einsatz quartärer, organischer Ammoniumsalze, besonders bevorzugt Tetra-n-butylammoniumsalze, N-Cetyl-N,N,N-trimethylammoniumsalze, Tetrabenzylammoniumsalze oder Triethylbenzylammoniumsalze.

Ganz besonders bevorzugt werden die quartären, organischen Ammoniumsalze in situ in der Reaktionsmischung erzeugt, indem der Mischung tertiäre Amine der Struktur 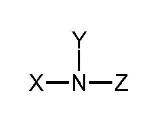 worin
- X, Y und Z: gleich oder ungleiche aliphatische Reste sind,
zugegeben werden. Diese Amine reagieren mit dem Benzylhalogenid zu den katalytisch wirksamen quartären, organischen Ammoniumhalogeniden. Es wird aus Kostengründen vorgezogen, dass X, Y und Z Alkylreste sind. Nichteinschränkende Beispiele solcher trialiphatischer Amine sind Trimethylamin, Triethylamin, Tripropylamin, Tri-n-butylamin, Triisoamylamin, Trihexylamin, Methyldiethylamin, Dimethylethylamin, Dimethylcyclohexylamin, Dimethylhexylamin, Benzyldimethylamin, Diethylhexylamin, Diemethyldecylamin. Unter den aromatischen Aminen sind besonders Pyridinderivate und Imidazolderivate wie Methylpyridin, N-Methylimidazol, insbesondere aber die Grundkörper zu nennen.

Schließlich eignen sich auch Kronenether, wie zum Beispiel 18-Krone-6, und Polyethylenglycole mit Molekulargewichen zwischen 200 bis 4000 g/mol zur Phasentransferkatalyse für die Herstellung oben genannter Ester.

Die Erfindung umfasst auch die Verwendung der Estermischungen als Weichermacher für Kunststoffe bevorzugt für Polyvinylchlorid, Vinylchlorid-basierte Copolymere, Polyvinylidenchlorid, Polyvinylacetale, Polyacrylate, Polyamide, Polylactide, Cellulose und seine Derivate, Kautschukpolymere wie Acryl-nitril-Butadien-Kautschuk, hydrierten Acrylnitril-Butadien-Kautschuk, Chloroprenkautschuk, chloriertes Polyethylen, Chlorsulfonylpolyethylen, Ethylen-Propylen-Kautschuk, Acrylatkautschuk und/oder Epichlorhydrinkautschuk. Besonders bevorzugt ist die Verwendung als Weichmacher für Polyvinylchlorid.

Das Polyvinylchlorid wird dabei vorzugsweise durch Homopolymerisation aus Vinylchlorid nach dem Fachmann bekannten Methoden wie der Suspensions-, der Emulsions- oder der Massepolymerisation hergestellt. Die erfindungsgemäßen Estermischungen werden vorzugsweise in Gemischen mit 20 bis 99 % Polyvinylchlorid, bevorzugt 45 bis 95 % Polyvinylchlorid, besonders bevorzugt 50 bis 90 % Polyvinylchlorid eingesetzt. Diese Gemische werden Weich-Polyvinylchlorid genannt und können neben den erfindungsgemäßen Estermischungen und Polyvinylchlorid auch noch andere geeignete Zusatzstoffe enthalten. Beispielsweise können Stabilisatoren, Gleitmittel, Füllstoffe, Pigmente, Flammschutzmittel, Lichtstabilisatoren, Treibmittel, polymere Verarbeitungshilfsmittel, Schlagzähverbesserer, optische Aufheller, Antistatika oder Biostabilisatoren enthalten sein.

Die Kunststoffe erhalten vorzugsweise auch Zusatzstoffe wie Stabilisatoren, Gleitmittel, Füllstoffe, Pigmente, Flammschutzmittel, Lichtstabilisatoren, Treibmittel, polymere Verarbeitungshilfsmittel, Schlagzähverbesserer, optische Aufheller, Antistatika und/oder Biostabilisatoren sowie Mischungen davon.

Im Folgenden werden einige geeignete Zusatzstoffe näher beschrieben. Die aufgeführten Beispiele stellen jedoch keine Einschränkung der erfindungsgemässen Gemische dar, sondern dienen lediglich der Erläuterung. Alle Angaben zum Gehalt sind Gewichts-%-Angaben.

Stabilisatoren neutralisieren die während und/oder nach der Verarbeitung des Polyvinylchlorid abgespaltene Salzsäure. Als Stabilisatoren kommen alle üblichen Polyvinylchlorid-Stabilisatoren in fester und flüssiger Form in Betracht, beispielsweise übliche Epoxy/Zink-, Ca/Zn-, Ba/Zn-, Pb- oder Sn-Stabilisatoren sowie auch säurebindende Schichtsilikate wie Hydrotalcit. Die erfindungsgemäßen Estermischungen können in Gemischen mit einem Gehalt an Stabilisatoren von 0,05 bis 7 %, bevorzugt 0,1 bis 5 %, besonders bevorzugt von 0,2 bis 4 % und insbesondere von 0,5 bis 3 % eingesetzt werden.

Gleitmittel sollen zwischen den Polyvinylchlorid-Partikeln wirksam werden und Reibungskräften beim Mischen, Plastifizieren und Verformen entgegenwirken. Als Gleitmittel können die erfindungsgemässen Gemische alle für die Verarbeitung von Kunststoffen üblichen Gleitmittel enthalten. Beispielsweise kommen in Betracht Kohlenwasserstoffe, wie Öle, Paraffine und PE-Wachse (PE = Polyethylen), Fettalkohole mit 6 bis 20 C- Atomen, Ketone, Carbonsäuren, wie Fettsäuren und Montansäuren, oxidiertes PE-Wachs, Metallsalze von Carbonsäuren, Carbonsäureamide sowie Carbonsäureester, beispielsweise mit den Alkoholen Ethanol, Fettalkoholen, Glycerin, Ethandiol, Pentaerythrit und langkettige Carbonsäuren als Säurekomponente. Die erfindungsgemässen Estermischungen können in Gemischen mit einem Gehalt an Gleitmitteln von 0,01 bis 10 %, bevorzugt 0,05 bis 5%, besonders bevorzugt von 0,1 bis 3 % und insbesondere von 0,2 bis 2 % eingesetzt werden.

Füllstoffe beeinflussen vor allem die Druck-, Zug- und Biegefestigkeit sowie die Härte und Wärmeformbeständigkeit von weichgemachtem Polyvinylchlorid oder Polyvinylbromid in positiver Weise. Im Rahmen der Erfindung können die Gemische auch Füllstoffe wie beispielsweise Russ und andere anorganische Füllstoffe, wie natürliche Calciumcarbonate, beispielsweise Kreide, Kalkstein und Marmor, synthetische Calciumcarbonate, Dolomit, Silikate, Kieselsäure, Sand, Diatomeenerde, Aluminiumsilikate, wie Kaolin, Glimmer und Feldspat. Vorzugsweise werden als Füllstoffe Calciumcarbonate, Kreide, Dolomit, Kaolin, Silikate, Talkum oder Ruß eingesetzt. Die erfindungsgemässen Estermischungen können in Gemischen mit einem Gehalt an Füllstoffen von 0,01 bis 80 %, bevorzugt 0,1 bis 60 %, besonders bevorzugt von 0,5 bis 50 % und insbesondere von 1 bis 40 % eingesetzt werden.

Die mit den erfindungsgemässen Estermischungen zubereiteten Gemische können auch Pigmente enthalten, um das erhaltene Produkt an unterschiedliche Einsatzmöglichkeiten anzupassen. Im Rahmen der vorliegenden Erfindung können sowohl anorganische Pigmente als auch organische Pigmente eingesetzt werden. Als anorganische Pigmente können beispielsweise Cadmium-Pigmente, wie CdS, Kobalt-Pigmente, wie CoO/Al₂O₃, und Chrom-Pigmente, beispielsweise Cr₂O₃, verwendet werden. Als organische Pigmente kommen beispielsweise Monoazopigmente, kondensierte Azopigmente, Azomethinpigmente, Anthrachinonpigmente, Chinacridone, Phthalocyaninpigmente, Dioxazinpigmente und Anilinpigmente in Betracht. Die erfindungsgemäßen Estermischungen können in Gemischen mit einem Gehalt an Pigmenten von 0,01 bis 10 %, bevorzugt 0,05 bis 5 %, besonders bevorzugt von 0,1 bis 3 % und insbesondere von 0,5 bis 2 % eingesetzt werden.

Um die Entflammbarkeit zu vermindern und die Rauchentwicklung beim Verbrennen zu verringern, können die erfindungsgemäßen Gemische auch Flammschutzmittel enthalten. Als Flammschutzmittel können beispielsweise Antimontrioxid, Phosphatester, Chlorparaffin, Aluminiumhydroxid, Borverbindungen, Molybdäntrioxid, Ferrocen, Calciumcarbonat oder Magnesiumcarbonat verwendet werden. Die erfindungsgemäßen Estermischungen können in Gemischen mit einem Gehalt an Flammschutzmittel von 0,01 bis 30 %, bevorzugt 0,1 bis 25 %, besonders bevorzugt von 0,2 bis 20 % und insbesondere von 0,5 bis 15 % eingesetzt werden.

Um Artikel, die aus einem die erfindungsgemäßen Estermischungen enthaltenden Gemisch hergestellt wurden, vor einer Schädigung im Oberflächenbereich durch den Einfluss von Licht zu schützen, können die Gemische auch Lichtstabilisatoren enthalten. Es können im Rahmen der vorliegenden Erfindung beispielsweise Hydroxybenzophenone oder Hydroxyphenylbenzotriazole eingesetzt werden. Die erfindungsgemäßen Estermischungen können in Gemischen mit einem Gehalt an Lichtstabilisatoren von 0,01 bis 7 %, bevorzugt 0,1 bis 5 %, besonders bevorzugt von 0,2 bis 4 % und insbesondere von 0,5 bis 3 % eingesetzt werden.

Die Kunststoffe enthalten vorzugsweise auch weitere Weichmacher wie Monoalkylester der Benzoesäure, Benzoesäurediester von Mono-, Di-, Tri- oder Polyalkylenglycolen, Dialkylester aliphatischen Disäuren, Dialkylester aromatischer Disäuren, Trialkylester aromatischer Trisäuren, Phenylester von Alkansulfonsäuren, Alkyl- oder Arylester der Phosphorsäure, Polyester aus Dicarbonsäuren sowie Mischungen davon.

Beispiele für weitere Weichmacher sind
- die Monoalkylester der Benzoesäure, wie zum Beispiel Isononylbenzoat,
- die Benzoesäurediester von Mono-, Di-, Tri- oder Polyalkylenglycolen, wie zum Beispiel Propylenglycoldibenzoat, Diethylenglycoldibenzoat, Dipropylenglycoldibenzoat, Triethylenglycoldibenzoat oder Poly-ethylenglycoldibenzoat und insbesondere Mischungen davon,
- die Dialkylester aliphatischer Disäuren, wie zum Beispiel Di-(2-ethylhexyl)-adipat, Diisononyladipat, Di-(2-ethylhexyl)-sebazat, Di-(2-ethyl-hexyl)-azelat, Diisononyl-cyclohexan-1,2-dicarboxylat,
- die Dialkylester aromatischer Disäuren, wie zum Beispiel Di-(2-ethylhexyl)-phthalat, Diisononylphthalat, Diisodecylphthalat, Benzylbutylphthalat, Benzylisooctylphthalat, Benzylisononylphthalat,
- die Trialkylester aromatischer Trisäuren, wie zum Beispiel Trioctyltrimellitat,
- die Phenylester von Alkansulfonsäuren, wie zum Beispiel das Produkt Mesamoll^{®} der LANXESS Deutschland GmbH,
- die Alkyl- oder Arylester der Phosphorsäure, wie zum Beispiel Tri-(2-ethylhexyl)-phosphat, Diphenyl-2-ethylhexyl-phosphat, Diphenylkresylphosphat oder Trikresylphosphat,
- Polyester, die zum Beispiel aus Dicarbonsäuren wie Adipinsäure oder Phthalsäure und Diolen wie 1,2-Propandiol, 1,3-Butandiol, 1,4-Butandiol oder 1,6-Hexandiol hergestellt werden können.

Im Rahmen der Erfindung können die erfindungsgemässen Estermischungen auch in Gemischen eingesetzt werden, die weitere Kunststoffe ausgewählt aus der Gruppe bestehend aus Homo- und Copolymeren auf Basis von Ethylen, Propylen, Butadien, Vinylacetat, Glycidylacrylat, Glycidylmethacrylat, Acrylaten und Methacrylaten mit Alkoholkomponenten von verzweigten oder unverzweigten C₁- bis C₁₀-Alkoholen, Styrol oder Acrylnitril enthalten. Zu nennen sind beispielsweise Polyacrylate mit gleichen oder verschiedenen Alkoholresten aus der Gruppe der C₄-bis C₈-Alkohole, besonders des Butanols, Hexanols, Octanols und 2-Ethylhexanols, Polymethylmethacrylat, Methyl-methacrylat-Butylacrylat-Copolymere, Methylmethacrylat-Butylmethacrylat-Copolymere, Ethylen-Vinylacetat-Copolymere, chloriertes Polyethylen, Nitrilkautschuk, Acrylnitril-Butadien-Styrol-Copolymere, Ethylen-Propylen-Copolymere, Ethylen-Propylen-Dien-Copolymere, Styrol-Acrylnitril-Copolymere, Acrylnitril-Butadien-Rubber, Styrol-Butadien-Elastomere und Methyhnethacrylat-Styrol-Butadien-Copolymere.

Die mit den erfindungsgemäßen Estermischungen hergestellten Gemische sind beispielsweise nützlich zur Herstellung von Rohrleitungen, Kabeln, Draht-Ummantelungen, im Innenausbau, im Fahrzeug- und Möbelbau, in Bodenbelägen, medizinischen Artikeln, Lebensmittelverpackungen, Dichtungen, Folien, Verbundfolien, Folien für Verbundsicherheitsglas, insbesondere für den Fahrzeug-Bereich und den Architektur-Sektor, Kunstleder, Spielzeug, Verpackungsbehältern, Klebebandfolien, Bekleidung, Beschichtungen, sowie Fasern für Gewebe.

Die erfindungsgemässen Estermischungen sind wegen ihrer sehr niedrigen Lösetemperaturen gut verarbeitbar und weisen eine geringe Flüchtigkeit auf.

Die Weichmacher und das Verfahren dieser Erfindung werden durch die nachfolgenden den Erfindungsbereich nicht einschränkenden Beispiele erläutert. [Angaben in Flächenprozenten].

### Beispiele

### Bestimmung der Lösetemperatur

Zur Bestimmung der Lösetemperatur wurden 48 g des zu prüfenden Weichmachers im Becherglas mit 2 g Polyvinylchlorid, Typ Vinnol^{®} H70, Korngröße < 315 µm, und 2 Tropfen Irgastab 17 M verrührt. Die Suspension wurde unter Rühren mit 2 - 3 °C pro Minute solange aufgeheizt, bis eine Temperatur erreicht ist, bei der 3 Minuten hintereinander keinerlei Anstieg in der Anzeige einer hinter dem Becherglas positionierten Photozelle mehr zu verzeichnen ist und das Polyvinylchlorid gelöst ist.

### Bestimmung der Flüchtigkeit

Als Maß für die Flüchtigkeit wurde die Menge der kondensierbaren Bestandteile wie folgt bestimmt. Dabei werden 10 g des Weichmachers in ein thermostatiertes, zylindrisches Gefäß gegeben (Fogging Test Gerät N8-FPG). Als Kondensationsflächen dient eine gekühlte Aluminiumfolie, deren Gewicht zuvor bestimmt wurde. Anschließend wird der versiegelte Zylinder für 16 h auf 100°C erwärmt. Dann wird die Aluminiumfolie entnommen. Nach Lagerung im Exikator für 4 h wird durch Differenzwägung die Gewichtszunahme ermittelt. Die Flüchtigkeit wird durch Doppelbestimmung ermittelt.

### Beispiele 1 bis 5: Herstellung der Estermischungen

In einem 2000 ml Vierhalsrundkolben mit Rückflusskühler, Innenthermometer, KPG-Rührer wurden Benzylchlorid, n-Butanol, Triethylamin und wasserfreies Natriumcarbonat in einer Stickstoffatmosphäre vorgelegt. Anschließend wurde die Reaktionsmischung auf 90 °C erwärmt und Trimellitsäureanhydrid innerhalb von 3,5 h portioniert zugegeben. Anfänglich wird eine starke Gasentwicklung bei den Trimellitsäurezugaben beobachtet. Anschließend wird für 8 h bei 90 °C gerührt. Nun folgen drei Wäschen mit Wasser bei 80 °C (Wasser/Organik: 1/1). Dabei befindet sich die organische Phase in der ersten Wäsche oben, in den darauf folgenden Wäschen unten. Anschließend wird eine Wasserdampfdestillation durchgeführt, bis das Zweifache des Volumens bezogen auf organisches Rohprodukt an Kondensat aufgefangen wird. Schließlich erfolgt die Trocknung bei 140 °C und 4 mbar am Rotationsverdampfer. Man erhält eine farblose viskose Flüssigkeit. Zusammensetzung und Ausbeuten sind der nachfolgenden Tabelle zu entnehmen.

**Tabelle 1**

| **Beispiel** | **1** | **2** | **3** | **4** | **5** |
|---|---|---|---|---|---|
| n-BuOH (mol) | 1,08 | 1,05 | 1,10 | 1,20 | 1,20 |
| Benzylchlorid (mol) | 2,20 | 2,20 | 2,05 | 2,05 | 2,05 |
| Na₂CO₃(mol) | 1,33 | 1,33 | 1,33 | 1,33 | 1,33 |
| Net₃(mol% auf TMA) | 0,04 | 0,04 | 0,07 | 0,07 | 0,06 |
| SZ[mgKOH/g] | < 0,1 | < 0,1 | < 0,1 | < 0,1 | < 0,1 |
| Ausbeute [%] | 75 | 73 | n.d. | 75 | 82 |

| **Zusammensetzung %** | | | | | |
|---|---|---|---|---|---|
| Tributyltrimellitat | 0 | 0 | 0 | 0 | 0 |
| Dibutylbenzyltrimellitat | 1,9 | 1,9 | 0,8 | 3,7 | 2,4 |
| Monobutyldibenzyltrimellitat | 89,6 | 75,7 | 85,0 | 79,3 | 83,2 |
| Tribenzyltrimellitat | 6,9 | 18,5 | 11,2 | 11,9 | 8,4 |

| | | | | | |
|---|---|---|---|---|---|
| n.d.: nicht bestimmt; TMA: Trimellitsäureanhydrid; Zusammensetzung in GC%. | | | | | |

### Stoffeigenschaften der Estermischung aus Beispiel 1

**Tabelle 2**

| **Beispiel** | **Lösetemperatur (°C)** | **Viskosität (mPas)** | **Pour-Point (°C)** | **Kondensat (mg)** |
|---|---|---|---|---|
| 1 | 116 | 832 | -10 | 13 |

| **Nicht erfindungsgemäß:** | | | | |
|---|---|---|---|---|
| Unimoll® BB | 110 | 62 | -40 | 36 |
| Uraplast® 525 | 160 | 114 | -43 | 0,13 |

| | | | | |
|---|---|---|---|---|
| Unimoll^{®} BB: Benzylbutylphthalat;. Uraplast^{®} 525: C₈-C₁₀-Trialkyltrimellitat von linearen C₈- bis C₁₀-Alkoholen. | | | | |

Aus den Beispielen ist leicht zu ersehen, dass das beschriebene Verfahren in hohen Ausbeuten unter Vermeidung von stöchiometrischen Mengen tertiärer Amine die erfindungsgemäßen Weichmacher herzustellen erlaubt. Die Lösetemperatur der erfindungsgemäßen Weichmacher ist niedriger als die Lösetemperatur entsprechender reiner Trialkyltrimellitate und vergleichbar mit der von kommerziellen Benzylallcylphthalaten. Gleichzeitig zeigen die erfindungsgemäßen Weichmacher eine geringere Flüchtigkeit als korrespondierende kommerzielle Benzylalkylphthalate.

## Patentansprüche

1. Estermischung enthaltend
a) 80 - 99 Gewichts-% eines oder mehrerer Ester der Struktur (R¹OOC)ₙ-Z-(COOR²)p
b) 1-20 Gewichts-% eines Ester der Struktur (R²OOC)ₘ-Z
wobei die Summe der Gewichts-% aus den Komponenten der Estermischungen 100 ergibt und
worin
R¹ ein geradkettiger oder verzweigter C₁- bis C₂₀-Alkylrest,
R² ein optional mit C₁- bis C₄-Alkyl oder Halogen substituierter Benzylrest,
Z ein m-wertiger aromatischer C₆- bis C₁₀-Kohlenwasserstoffrest,
m eine Zahl von 3 bis 4 und
n und p jeweils eine Zahl von 1 bis 3 unter der Maßgabe n + p = m
bedeuten.

2. Estermischung nach Anspruch 1, **dadurch gekennzeichnet, dass** R² für Benzyl steht.

3. Estermischung nach Anspruch 1 und/oder 2, **dadurch gekennzeichnet, dass** R¹ für n-Butyl, 2-Ethylhexyl oder Isononyl steht.

4. Estermischung nach einem der Ansprüche 1 bis 3, **dadurch gekennzeichnet, dass** es sich bei den Estern der Struktur (R¹OOC)ₙ-Z-(COOR²)ₚ um Benzyldibutyltrimellitat und Dibenzylbutyltrimellitat und bei dem Ester der Struktur (R²OOC)ₘ-Z um Tribenzyltrimellitat handelt.

5. Verfahren zur Herstellung von Estermischungen enthaltend
a) 80 - 99 Gewichts-% eines oder mehrere Ester der Struktur (R¹OOC)ₙ-Z-(COOR²)ₚ
b) 1-20 Gewichts-% eines Ester der Struktur (R²OOC)ₘ-Z
wobei die Summe der Gewichts-% aus den Komponenten der Estermischungen 100 ergibt und
worin
R¹ ein geradkettiger oder verzweigter C₁- bis C₂₀-Alkylrest,
R² ein optional mit C₁- bis C₄-Alkyl oder Halogen substituierter Benzylrest,
Z ein m-wertiger aromatischer C₆- bis C₁₀-Kohlenwasserstoffrest,
m eine Zahl von 3 bis 4 und
n und p jeweils eine Zahl von 1 bis 3 unter der Maßgabe n + p = m
bedeuten, **dadurch gekennzeichnet, dass** mindestens eine aromatische drei- oder vierbasige Polycarbonsäure oder ein Derivat davon, mindestens ein Benzylhalogenid und mindestens ein aliphatischer Alkohol bei Temperaturen von 50 bis 300 °C und Drücken von 2 mbar bis 10 bar in Gegenwart einer anorganischen Base und einer substöchiometrischen Menge eines Phasentransferkatalysators miteinander umgesetzt werden.

6. Verfahren nach Anspruch 5, **dadurch gekennzeichnet, dass** Polycarbonsäuren oder deren Derivate ausgewählt aus der Gruppe bestehend aus Pyromellithsäure oder Trimellitsäure, eingesetzt werden.

7. Verfahren gemäß Anspruch 6, **dadurch gekennzeichnet, dass** als Derivate die Anhydride eingesetzt werden.

8. Verfahren gemäß Ansprüche 5 bis 7, **dadurch gekennzeichnet, dass** unsubstituiertes Benzylchlorid und als aliphatischer Alkohol n-Butanol, 2-Ethylhexanol oder Isononanol eingesetzt werden.

9. Verwendung der Estergemische gemäß den Ansprüchen 1 bis 4 als Weichermacher für Kunststoffe.

10. Verwendung gemäß Anspruch 9, **dadurch gekennzeichnet, dass** es sich bei den Kunststoffen um Polyvinylchlorid, Vinylchlorid-basierte Copolymere, Polyvinylidenchlorid, Polyvinylacetale, Polyacrylate, Polyamide, Polylactide, Cellulose und seine Derivate oder Kautschukpolymere, bevorzugt Acrylnitril-Butadien-Kautschuk, hydrierter Acrylnitril-Butadien-Kautschuk, Chloroprenkautschuk, chloriertes Polyethylen, Chlorsulfonylpolyethylen, Ethylen-Propylen-Kautschuk, Acrylatkautschuk und/oder Epichlorhydrinkautschuk handelt.

11. Verwendung der Estermischungen gemäß der Ansprüche 9 oder 10, **dadurch gekennzeichnet, dass** die Kunststoffe Zusatzstoffe der Reihe Stabilisatoren, Gleitmittel, Füllstoffe, Pigmente, Flammschutzmittel, Lichtstabilisatoren, Treibmittel, polymere Verarbeitungeshilfsmittel, Schlagzähverbesserer, optische Aufheller, Antistatika und/oder Biostabilisatoren sowie Mischungen davon enthalten.

12. Verwendung der Estermischungen gemäß Anspruch 11, **dadurch gekennzeichnet, dass** die Kunststoffe auch weitere Weichmacher, bevorzugt Monoalkylester der Benzoesäure, Benzoesäurediester von Mono-, Di-, Tri- oder Polyalkylenglycolen, Dialkylester aliphatischer Disäuren, Dialkylester aromatischer Disäuren, Trialkylester aromatischer Trisäuren, Phenylester von Alkansulfonsäuren, Alkyl- oder Arylester der Phosphorsäure, Polyester aus Dicarbonsäuren sowie Mischungen davon enthalten.
